# EUROPEAN PATENT APPLICATION

(11) **EP 3 594 364 A1**
(43) Date of publication of application: **15.01.2020**
(21) Application number: 18183542.2
(22) Date of filing: 13.07.2018
(51) Int. Cl.: C12Q 1/6806, C12Q 1/6869

(54) **METHOD OF ASSAYING NUCLEIC ACID IN MICROFLUIDIC DROPLETS**

(71) Applicant: HiFiBio SAS High Fidelity Biology, 75014 Paris (FR)
(72) Inventor: Grosselin, Kevin Armand, 75014 Paris (FR); Gérard, Annabelle Patricia Véronique, 91120 Palaiseau (FR)
(74) Representative: CH Kilger Anwaltspartnerschaft mbB

(57) **Abstract**

A first aspect of the present invention relates to a method of identifying one or more genomic region(s) of interest using a microfluidic system, the method comprising the steps of:
a. providing at least a droplet of first type, wherein said droplet of first type comprises
i. a biological element,
ii. a lysis buffer, and
iii. a nuclease,

b. collecting said droplet of first type under conditions that temporarily inactivate said nuclease,
c. incubating said droplet of first type, thereby reactivating said nuclease,
d. providing at least a droplet of second type, wherein said droplet of second type comprises a nucleic acid sequence,
e. merging said droplets of first and second type, thereby generating a droplet of third type,
f. incubating said droplet of third type, thereby linking said nucleic acid sequence to at least one genomic region(s) of interest,
g. sequencing said one or more genomic region(s) of interest.

A second aspect of the present invention relates to a nucleic acid sequence comprising:
a. at least one index sequence,
b. a sequencing adaptor, and
c. at least one protecting function positioned at 3'- and/or 5'-end.

## Description

### FIELD OF THE INVENTION

The present invention is in the field of molecular biology and microfluidics. Particularly, the invention relates to a method of assaying nucleic acid in microfluidic droplets. The invention also encompasses a nucleic acid sequence/structure for use in profiling single cell epigenetic and transcriptomic state in a biological sample.

### BACKGROUND OF THE INVENTION

Eucaryotic genomes are organized into chromatin which enables not only to compact DNA but also regulates DNA metabolism (replication, transcription, repair, recombination). A current challenge is thus to understand (i) how functional chromatin domains are established in the nucleus, (ii) how chromatin structure/information is dynamic through assembly, disassembly, modifications and remodeling mechanisms and (iii) how these events participate/maintain in disease establishment, progression and relapse of disease. Understanding these events will allow identifications of novel mechanisms of disease progression and new therapeutics targets, as well as controlling effect of therapeutics molecules.

In addition, a fundamental research question in biology is to understand how hundreds of distinct cell types arise from identical genetic material in multicellular organisms. The many different cell types can't be explained solely by genetics but rather by an additional information that can bridge phenotype to genotype. In 1942, Conrad H. Waddington coined the term Epigenetics as "the branch of biology which studies the causal interactions between genes and their products, which bring the phenotype into being". This additional layer of "epigenetic information" is stored in the form of chemical modifications to both DNA and histone proteins that constitute the chromatin. Epigenetic mechanisms through chromatin modifications regulate gene expression and shape specific chromatin landscapes, which allow predictions to be made about cell type and tissue identity.

DNA and histone modifications are involved in various DNA-based processes by serving as recognition sites for effector proteins capable of reading information and by stabilizing their binding to the chromatin. The large abundance of histone modifications enables a tight control of chromatin structure and a great flexibility in the regulation of DNA-based processes. This diversity leads to crosstalk between histones that can be modified at different sites simultaneously (Wang et al. 2008, Nature Genetics 40(7):897-903).

Histone modifications can positively or negatively affect each other. In addition, communication between histone modifications also exists with other chromatin modifications such as DNA methylation, which all participate to fine-tune the overall regulation of the biological functions (Du et al. 2015, Nature Reviews Molecular Cell Biology, 16(9):519-532).

DNA and histone modifications contribute to the definition of epigenomic signatures within distinct chromatin states, which are highly indicative of cell type and tissue identity. The genome-wide profiling of these marks can be leveraged to understand the global landscape of genome regulation and then, for example, distinguish epigenomic differences in the context of normal and disease cell states (Consortium Epigenomics 2015, Nature 518(7539):317-329). However, the current state of the chromatin profiling technologies does not allow studying cellular heterogeneity nor detect cell-to-cell variation in chromatin states.

Genome-wide mapping of epigenetic modifications, epigenetic markers/erasers, factors playing a role in chromatin structure, organization in 2D and 3D using traditional ChIP-seq method requires a large number of cells to generate high quality binding site profiles. Several studies have shown optimized ChlP-seq protocols to reduce input material from millions to hundreds of cells without losing resolution in the detection of enriched or depleted regions (Adli et al. 2010, Nature Methods 7(8):615-618; Brind'Amour et al. 2015, Nature Communications 6:6033; Ma et al. 2018, Science Advances 4(4):eaar8187). However, these methods only yield an averaged snapshot of the modification status, without providing insight into the epigenetic heterogeneity.

Profiling histone modifications at single-cell resolution remains challenging, in part because the level of noise associated with non-specific binding during the immunoprecipitation tends to increase with low quantity of starting material. Immunoprecipitating chromatin from one single cell is technically feasible but would lead to highly variable results.

Chromatin from isolated single cells can be indexed beforehand with a specific and unique DNA sequence (barcode), and then combined with indexed chromatin from a couple to several thousands of cells to perform immunoprecipitation in bulk as in traditional ChIP-seq protocol. This method circumvents the issue associated with high experimental noise in the immunoprecipitation of low input material, while retaining the single-cell information. Indeed, barcodes being specific of one cell, each read can be attributed to its originating cell after sequencing. However, like other single-cell technologies in which molecular indexing is involved, only indexed nucleosomes have the potential to be amplified and sequenced.

In this regard, Rotem developed a Drop-ChIP technology that combines chromatin indexing method with droplet-based microfluidics to profile histone modifications of thousands of cells (Rotem et al. 2015, Nature Biotechnol. 33(11):1165-1172). The droplet format provides a versatile tool for performing single-cells assays. Following the steps of compartmentalization, lysis and chromatin fragmentation by microccocal nuclease of the cell in droplets, said droplets are then merged one-to-one with a second population of droplets containing DNA barcodes, allowing chromatin indexing at the single-cell level.

Although Drop-ChIP was used to reveal distinct chromatin states within a population of embryonic stem cells, the single-cell information was limited to as few as hundreds of unique enriched loci detected per cell due to low chromatin indexing efficacy or poor recovery of indexed nucleosomes. Notably, Drop-ChIP technology suffers from two major limitations, which may negatively impact the amount of information recovered per cell. Firstly, only symmetrically indexed nucleosomes can be amplified and can be part of the sequencing library. This requirement dramatically increases the stringency of the system and imposes a strong selection on the nucleosomes (i.e. only those with both ends ligated to a barcode). Secondly, amplification of indexed nucleosomes only relies on numerous cycles of Polymerase Chain Reaction (PCR), which increases the probability to introduce amplification bias and errors.

In view of the above limitations affecting methods known in the art, it is evident that an improved method for single-cell epigenetic profiling in microfluidic droplets is required.

### SUMMARY OF THE INVENTION

A first aspect of the present invention relates to a method of identifying one or more genomic region(s) of interest using a microfluidic system, the method comprising the steps of:
a. providing at least a droplet of first type, wherein said droplet of first type comprises
   i. a biological element,
   ii. a lysis buffer, and
   iii. a nuclease,
b. collecting said droplet of first type under conditions that temporarily inactivate said nuclease,
c. incubating said droplet of first type, thereby reactivating said nuclease,
d. providing at least a droplet of second type, wherein said droplet of second type comprises a nucleic acid sequence,
e. merging said droplets of first and second type, thereby generating a droplet of third type,
f. incubating said droplet of third type, thereby linking said nucleic acid sequence to at least one genomic region(s) of interest,
g. sequencing said one or more genomic region(s) of interest.

A second aspect of the present invention relates to a nucleic acid sequence comprising:
a. at least one index sequence,
b. a sequencing adaptor, and
c. at least one protecting function positioned at 3'- and/or 5'-end.

### BRIEF DESCRIPTION OF THE DRAWINGS

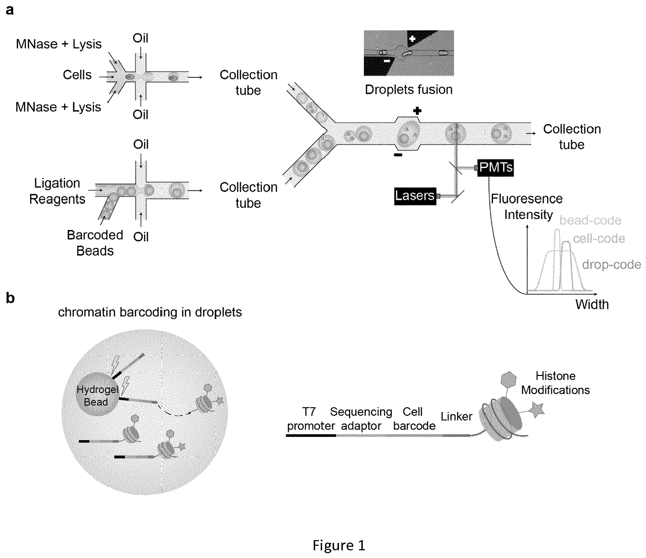

Figure 1 depicts the microfluidic workflow according to the present invention. (A) Cells are compartmentalized in 45pl droplets with the reagents necessary for lysis and chromatin fragmentation. In parallel, hydrogel beads carrying DNA barcodes are encapsulated in 100pl droplets with ligation reagents. The two emulsions are reinjected in a fusion device, the barcode drops (100pl) and the nucleosome drops (45pl) are paired asymmetrically and an electric field triggers the fusion. Fused droplets are scanned one-by-one by a laser beam and the composition of each droplet is analyzed in real time. (B) Emulsion of fused droplets is collected for nucleosomes barcoding in droplets. Barcodes are released from the beads by photo-cleavage and ligated to the nucleosomes. The content of droplets is combined, immunoprecipitated and the enriched DNA is sequenced. The deconvolution of the barcode associated reads attributes all sequences to their originating cell to reconstruct single-cell chromatin profile.

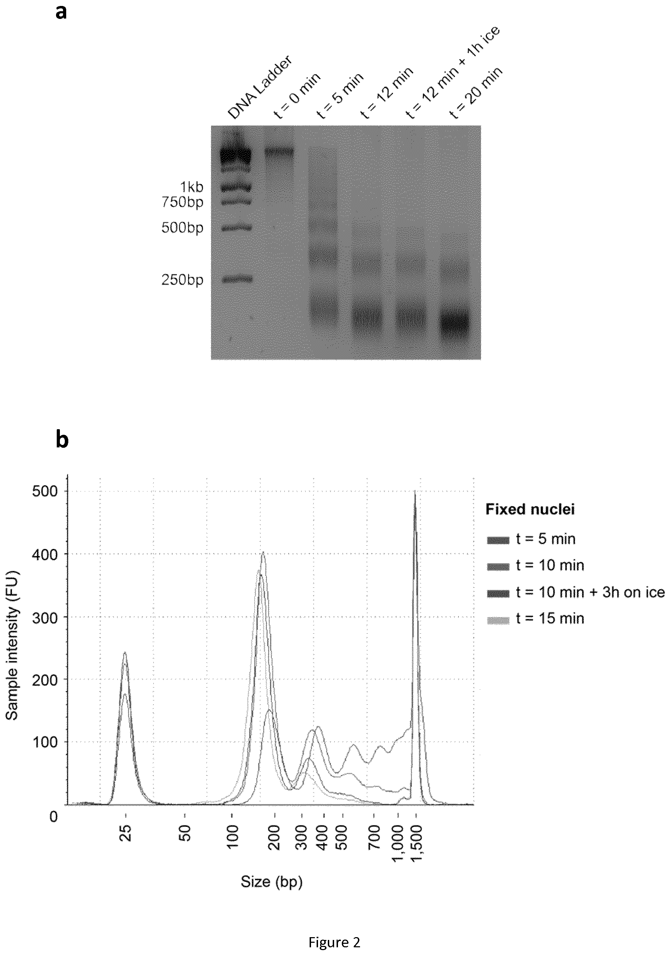

Figure 2 depicts the synchronization and pause of the MNase activity between droplets. Particularly, Figure 2a shows gel electrophoresis of DNA fragments from human Jurkat T cell at different time point of MNase incubation. At = 0min, DNA is not fragmented yet, confirming that MNase activity is synchronized upon droplets collection on ice. Time point t = 12min + 1h ice shows similar digestion profile as after 12min of incubation, confirming that MNase activity is paused when droplets are stored on ice. Figure 2b depicts the pause of MNase activity in droplets starting from fixed nuclei. MNase is paused for 3h without chromatin over-digestion.

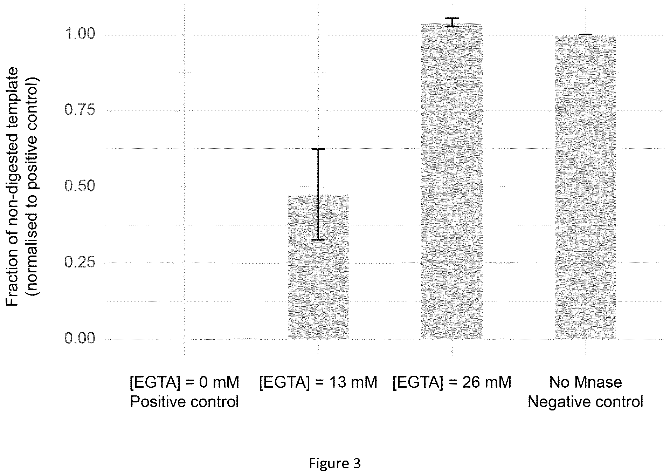

Figure 3 depicts the concentration of EGTA necessary to fully inactivates MNase activity in droplets. The fraction of oligonucleotide remaining after incubation was measured by TapeStation and normalized to the digestion negative control (i.e droplets without MNase). 26 mM final concentration of EGTA fully inactivated MNase in droplets. Barplot shows the mean fraction of non-digested oligonucleotides for duplicates and the error bars correspond to the standard deviation.

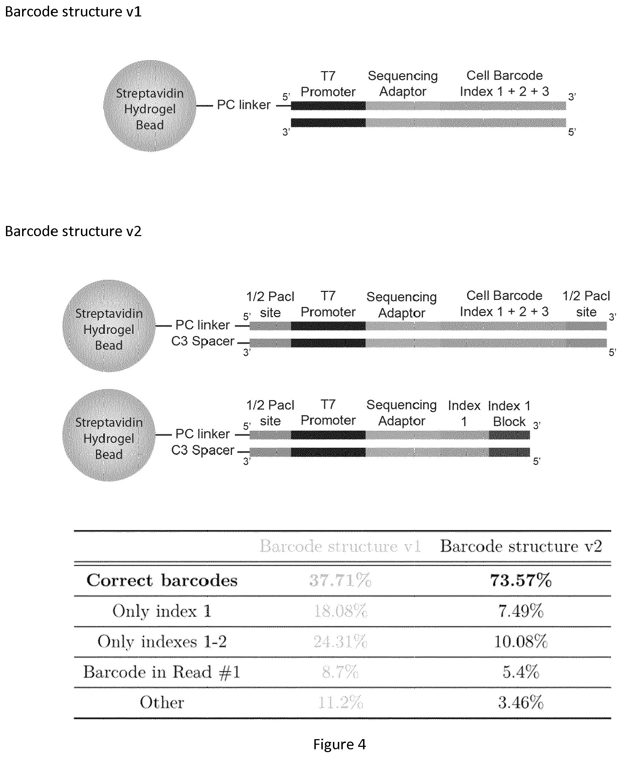

Figure 4 depicts the nucleic acid sequence according to the invention. The new structure (v2) allows digestion of barcode concatemers by addition of one half of Pac1 restriction site on both ends of the barcodes. A C3-spacer at the 3'-end of the bottom strand is also added to enforce the direction of the ligation. Non-full-length barcodes are completed with a protecting group containing modified bases modifications that prevent unwanted ligation. The table depicts the proportion of correct barcodes identified post-sequencing with each of the barcode structure.

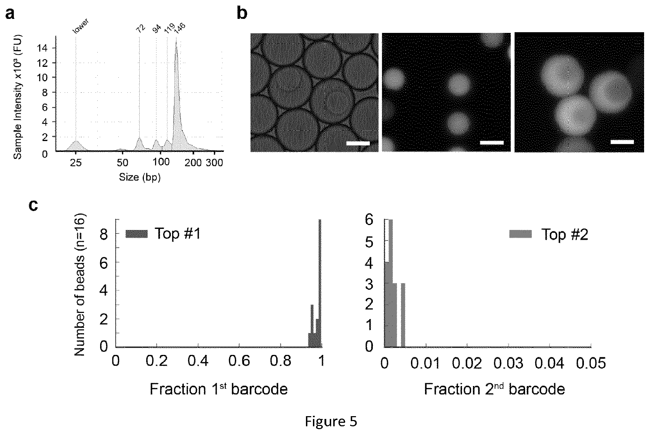

Figure 5 depicts the quality control of barcoded hydrogel beads. (a) Tapestation profile of DNA barcodes after photo-cleavage from hydrogel beads shows the presence of full-length barcodes (larger peak at 146 bp), as well as intermediates that failed to be completed (peaks at 72 bp, 94 bp and 119 bp). (b) Imaging of hydrogel beads in droplets using epifluorescence microscopy. From left to right: (i) bright field image; (ii) imaging after hybridization of a DNA probe complementary to the Illumina sequencing adaptor onto the barcodes; (iii) same as (ii) after release of the barcodes in droplets by photo-cleavage. Scale bars are 35 µm. (c) Single bead deep sequencing results showing the fraction of the first-two most abundant barcodes of 16 beads. In average, 97.7% of the barcodes present on a bead match to the same sequence while the second most abundant barcode represents only 0.17% of all sequencing reads.

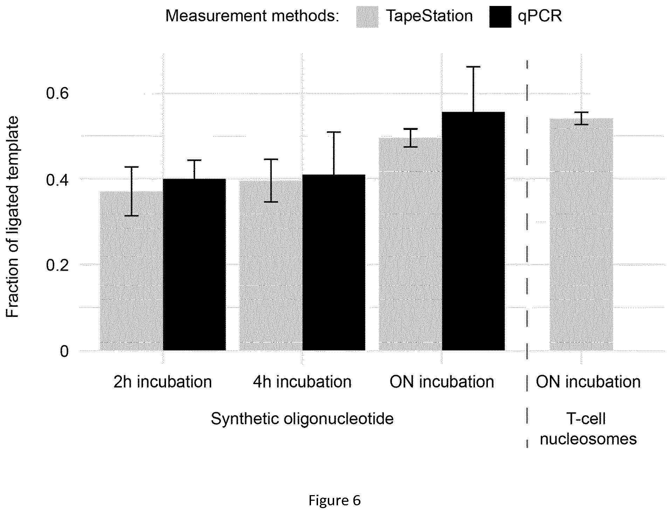

Figure 6 depicts the estimation of the ligation efficiency in droplets. The ligation was performed for 2h, 4h and overnight incubation. Both measurement methods gave similar estimation of the fraction of ligated product and a significant increase in efficiency was observed after an overnight incubation (∼10%). Barplot shows the mean fraction of ligated oligonucleotides for duplicates (experiments performed on two different days) and the error bars correspond to the standard deviation.

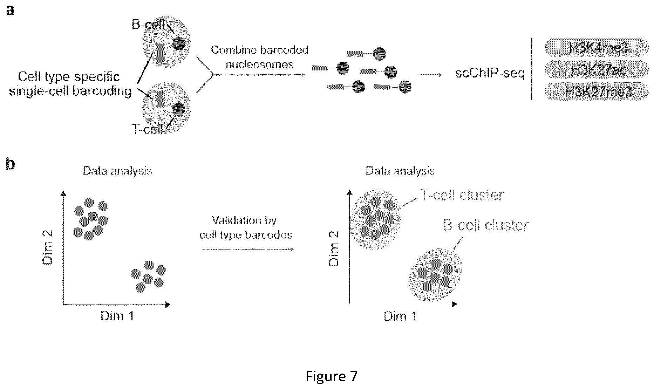

Figure 7 depicts the proof of concept study and expected outcomes. (a) Human B and T lymphocytes were separately encapsulated and specifically indexed in droplets. Indexed chromatin from the two emulsions were then combined for ChlP-seq targeting 3 distinct histone modifications (H3K4me3, H3K27ac and H3K27me3). (b) Data analysis would group cells into two clusters using an unsupervised clustering approach. The correct clustering of B and T-cells based on their chromatin profiles would then be confirmed by the cell type-specific barcode sequence.

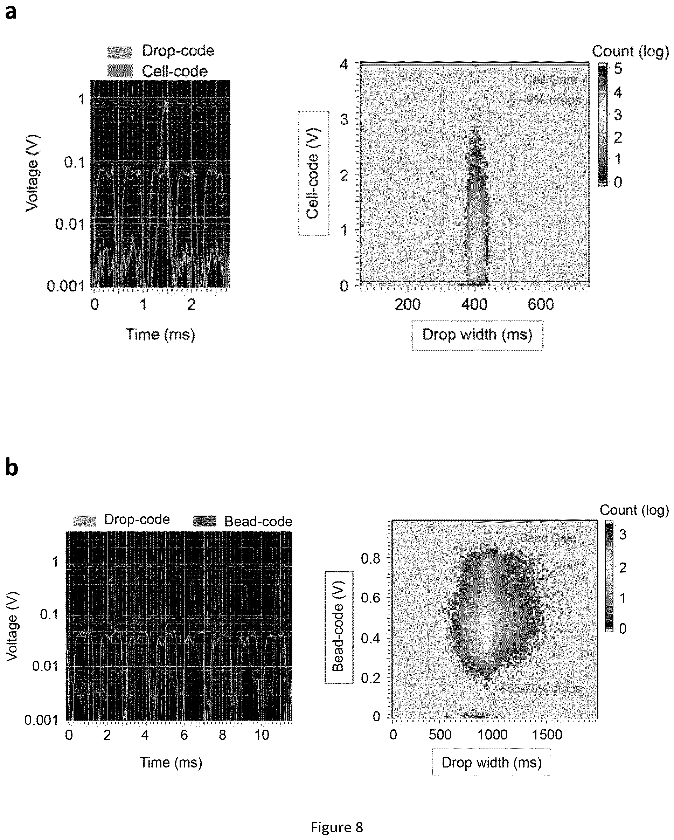

Figure 8 depicts monitoring of cells and hydrogel encapsulation in droplets. (a) Experimental time trace recorded for droplets analyzed at 1.8 kHz. Orange fluorescence is present in all droplets and used to control for the size of the drops (drop-code). Green fluorescence indicates the presence of a cell in a droplet (cell-code). Plot of cell-code intensity (green) vs drop-code intensity (orange) in each droplet. Droplets containing a cell have a high green cell-code intensity allowing counting of encapsulated cells with the definition of a cell-gate above the noise level. A cell density adjusted to λ = 0.1 results in ∼9% of the droplets containing one single cell. (b) Experimental time trace recorded for 100 pl droplets analyzed at 650 Hz. Orange fluorescence is present in all droplets and used as a drop-code to control for the size of the droplets. Red fluorescence indicates the presence of a hydrogel bead in a droplet (bead-code). Plot of bead-code intensity (red) versus drop-code intensity (orange) in each droplet. By using a close-packed ordering of the beads, 65% to 75% of the droplets contain one bead.

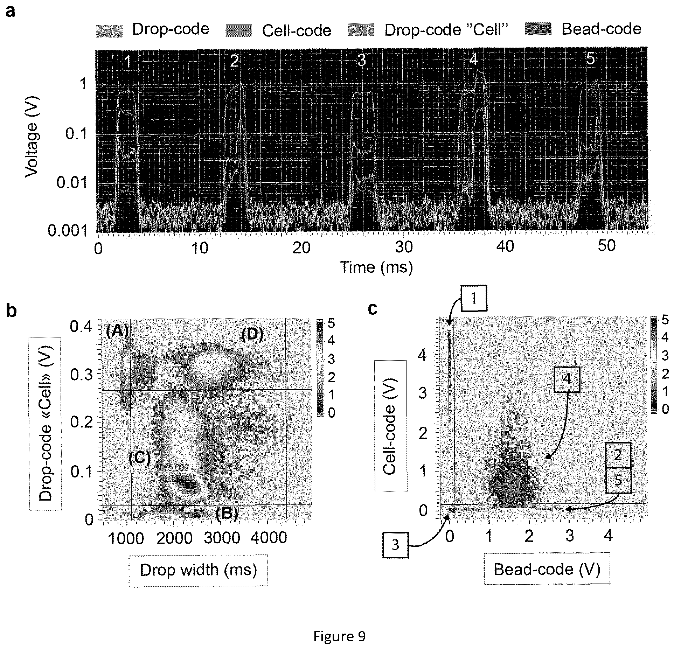

Figure 9 depicts live monitoring of droplets fusion. (a) Experimental time trace recorded for droplets after fusion at 150 Hz. Orange fluorescence is present in all droplets and used as a drop-code to control for the size of the fused drops. Green fluorescence indicates the presence of a cell and the red fluorescence indicates the presence of a hydrogel bead. Blue fluorescence is a drop-code specific of the cell emulsion. (b) Plot of drop-code "cell" intensity versus drop-code intensity in each droplet defining 4 main populations of droplets after fusion. The main population in the middle represents droplets correctly paired and fused (70% to 80%). Unpaired droplets from the bead-emulsion are in the bottom population and unpaired droplets from the cell-emulsion with a high blue-fluorescence intensity are in the top-left population. The last population (top right) is associated with incorrectly-paired droplets containing 2 cell-droplets fused with one bead-droplet. (c) Plotting cell-code intensity versus bead-code intensity in each droplet enables precise counting of useable drops (those containing one cell and one bead). Droplets from the time trace in panel (a) are indicated as example of the different populations.

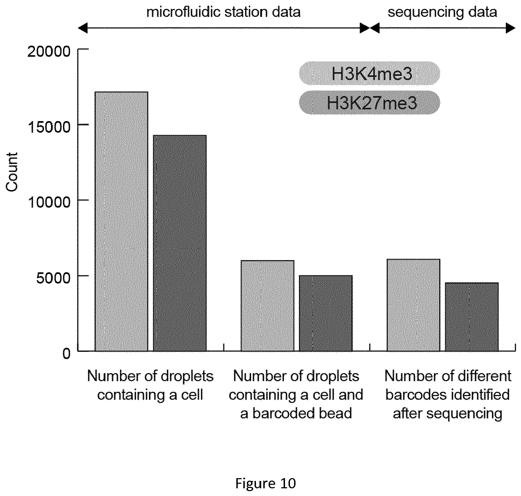

Figure 10 depicts the total number of cells and the number of cells co-encapsulated with a barcoded hydrogel bead detected by fluorescence on the microfluidic station in H3K4me3 and H3K27me3 single-cell ChlP-seq experiments. Analysis of sequencing data showed a number of identified barcodes closely related to the number of droplets containing both a cell and a bead counted on the microfluidic station, suggesting high overall efficacy of the system.

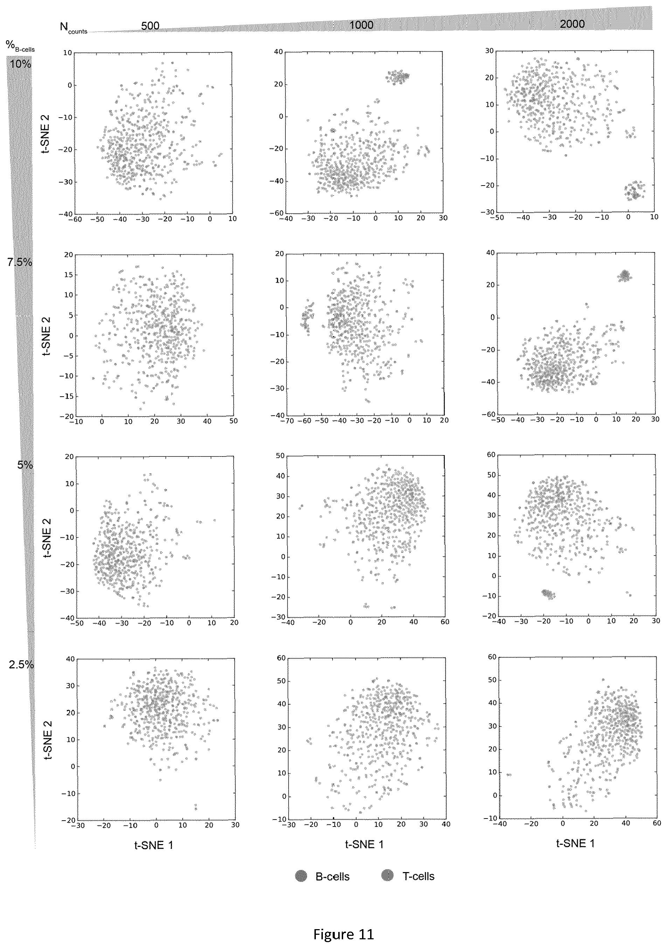

Figure 11 depicts the sensitivity of the scChIP-seq procedure to identify subpopulations. t-SNE plots representing H3K27me3 scChIP-seq dataset in in-silico simulation of detection limit with varying proportion of spiked-in B-cells in T-cells population (from top to bottom) and varying threshold of uniquely mapped reads per barcode (from left to right). Points are colored according to cell type specific barcode sequence.

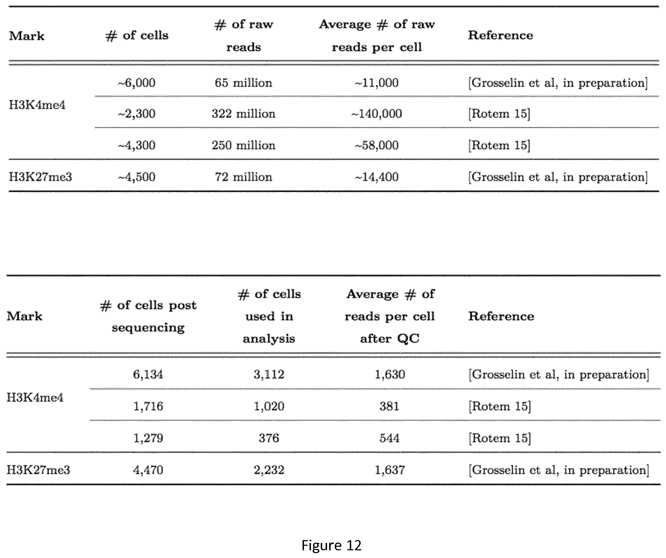

Figure 12 depicts sequencing performance in Drop-ChIP versus inventors' procedure. Table 1 compares the number of expected cells per sequencing library, the number of raw sequencing reads as well as the average number of raw reads per cell in Drop ChIP and in inventors' scChIP-seq system. Table 2 compares the number of cells identified after sequencing, the final number of cells used in analysis after QC and the average number of useable reads per cell after QC in Drop ChIP and in inventors' scChlP-seq system

### DETAILED DESCRIPTION SECTION OF THE INVENTION

The inventors have developed an improved single-cell ChIP method based on droplet microfluidics, which leads to a 5- to 10-fold increase in the number of enriched loci per individual cell compared to Drop-ChIP technology disclosed in Rotem (see Figure 7). The method allows with high sensitivity and precision to assess at the single cell level, histone modifications, DNA modified bases (including modified nucleotides for identifying ongoing DNA replication events at the single cell or any biological element level), chromatin/DNA associated factors. This method is applicable for the identifications of cell populations or any biological element having distinct features from another one, these features being histone and/or DNA modification, presence of factors. The presence or absence of these elements are then potentially indicative of variation in gene expression, and thus can be used as biomarkers, therapeutics targets to revert the changes.

It is well understood that cells can mean the nucleic, as compartment of chromatin structure. The cell or nuclei or any biological element can be fixed biological elements. Examples of fixative agents include aldehyde (including but not limited to formaldehyde, paraformaldehyde), alcohol (including but not limited to ethanol and methanol), oxidizing agents, mercurial, picrates, Hepes-glutamic acid buffer-mediated organic solvent protection effect (HOPE) fixatives.

As in Drop-ChIP, droplets containing cells and droplets containing barcodes are separately produced before being re-injected and merged one-to-one in a dedicated microfluidic fusion device (see Figure 1). However, the present method differs over Rotem in at least two aspects characterizing the barcode strategy. Firstly, the inventors replaced soluble barcodes emulsified from microtiter plates containing oligonucleotides by hydrogel beads (or any solid support) carrying millions of a unique or most abundant DNA sequence. Secondly, the novel design of the barcode structure according to the present invention allows linear amplification of all barcoded nucleosomes and not only symmetrically barcoded nucleosomes on both ends as in Rotem. Thirdly, the barcode design includes additional features increasing the efficiency of the barcoding of the nucleic acids of interest. These features include the addition of protective moiety to the 'incomplete barcode' preventing their attachment to the nucleic acid of interest. In another aspect, the barcode can include protectives bases on the full length oligos (protectives bases include, but are not limited to phosphorotioate, LNA/BNA, nucleotides phosphoramitidites, synthetic cycles, non 3'OH or 5'P bases, 2'-O-methyl-DNA/RNA), these protectives bases will protect the full length barcode while the non-full length barcode can be digested with exonuclease.

An additional set of barcodes, called 'experimental barcodes' can be added in order to multiplex different experiments in a single immuno-precipitation reaction. Subsequent bioinformatics analysis will allow to demultiplex the experimental conditions based on the sequence of the 'experimental barcode'.

It is well understood that the barcode is a nucleic acid sequence that can distinguish features specifics from nucleic acid originating from one compartment to another. Production of these barcodes are known by people skilled in the art and can represent a random sequence (flanked or not with known sequences), or generated by split pool synthesis (Klein et al., Cell, 2015).

Additionally, the method according to the present invention is characterized by a synchronization/pausing step, which limits cell-to-cell variation in chromatin digestion between droplets.

The aforementioned advantages are disclosed hereinafter in aspects and embodiments characterizing the present invention. Implementation of the invention is provided in examples and figures.

In a first aspect of the present invention, there is provided a method of identifying one or more genomic region(s) of interest using a microfluidic system, the method comprising the steps of:
a. providing at least a droplet of first type, wherein said droplet of first type comprises
   i. a biological element,
   ii. a lysis buffer, and
   iii. a nuclease,
b. collecting said droplet of first type under conditions that temporarily inactivate said nuclease,
c. incubating said droplet of first type, thereby reactivating said nuclease,
d. providing at least a droplet of second type, wherein said droplet of second type comprises a nucleic acid sequence,
e. merging said droplets of first and second type, thereby generating a droplet of third type,
f. incubating said droplet of third type, thereby linking said nucleic acid sequence to at least one genomic region(s) of interest,
g. sequencing said one or more genomic region(s) of interest.

The method of the present invention is carried out in a microfluidic system. In the context of the present invention, the term "microfluidic system" refers to a system or device having one or more channels and/or chambers that are generally fabricated on the micron or submicron scale.

The method of the present invention is characterized by the presence of droplets of first, second and third types. As used herein, the terms "first", "second" and "third" associated to droplets are used to discriminate droplets according to their content. As the method is performed in a microfluidic system, the term "droplet" also refers to "microfluidic droplet". Therefore, in the context of a microfluidic system, the term "droplet" also refers to an isolated portion of a first fluid that is surrounded by a second fluid, where the first and second fluids are immiscible.

According to the stages or step of the method of the present invention, the droplets may be contained in a microfluidic system (on-chip) or in a collector device (off-chip) separate from the microfluidic system. The droplets may have a spherical or non-spherical form.

In one embodiment of the present invention, the droplet has a volume ranging from about 20pl to about 100pl. Preferably, the droplet has a volume ranging from about 30pl to about 70pl. More preferably, the droplet has a volume ranging from about 40pl and about 50pl. Ideally, the droplet has a volume of about 45pl. As used herein, the term "about" refers to a range of values ±10% of a specified value.

As used herein, the term "lysis buffer" refers to buffer that is capable of lysing biological cells. The meaning of the term "lysis buffer" is within the common general knowledge of a person skilled in the art.

As used herein, the term "genomic region" refers to DNA or RNA encoded nucleic acid sequences.

As used herein, the term "nuclease" refers to an enzyme agent capable of cleaving a phosphodiester bond connecting nucleotide residues in a nucleic acid molecule. The nuclease may digest double-stranded, single-stranded, circular and linear nucleic acid molecules. In the context of the present invention, a nuclease may be an endonuclease, which cleaves a phosphodiester bonds within a polynucleotide chain, or an exonuclease, which cleaves a phosphodiester bond at the end of the polynucleotide chain, may be a transposase. A nuclease may also be a site-specific nuclease, which cleaves a specific phosphodiester bond within a specific nucleotide sequence, e.g. a recognition sequence. A non-limiting example of nuclease is Micrococcal nuclease (MNase). In a particular embodiment, the nuclease is a Micrococcal nuclease (MNase).

As used herein, the term "biological element" can refer to a single cell, a nuclei, a nucleic acid containing organelle (e.g. mitochondria) and can be obtained from an organism, a human or a non-human subject. In the latter case, the non-human subject is not limited to a mammal subject.

Performing enzymatic assays on individual biological element in droplets is challenging as cells are processed sequentially with different time scales. For example, the encapsulation step of the cells or any biological element lasts about 20min, which is in the same order of magnitude as the incubation step. Therefore, cells or any biological element encapsulated in droplets at the beginning will be longer in contact with nuclease than the cells or any biological element encapsulated in droplets at the end of the production. Similar observation can be made regarding the re-injection of the droplets in the fusion device (see general scheme in Figure 1). Indeed, the fusion of the two emulsions can last between 1h to 4h depending on the design of the experiment, meaning that some droplets containing fragmented DNA "wait" for hours before being fused and their MNase inactivated by EGTA. Consequently, synchronizing and pausing enzymatic activity are critical in order to avoid the introduction of chromatin digestion variation between individual cells or any biological element.

Notably, in conventional bulk ChlP-seq assays, the inactivation of a nuclease occurs immediately after nuclease incubation with addition of EGTA. Differently, in single-cell ChlP-seq assays, EGTA cannot be added immediately within the droplets and the nuclease is only inactivated after fusion with the barcodes-containing droplets.

In order to control and limit cell-to-cell or any biological element variation in chromatin digestion, the inventors introduced a step of collecting droplets of first type under conditions that temporarily inactivate said nuclease. Said collection step aimed to synchronize/pause the nuclease activity in the droplets is performed before each incubation step. The inventors identified that the droplet compartment render the MNase enzyme sensitive to temperatures changes and is capable of selectively blocking/reactivating and re blocking the enzyme activity. Such tight control of nuclease activity is not possible in bulk. This impact is suspected not being dependent on the sole MNase activity but to any enzyme.

Therefore, according to another embodiment, the method further comprises a step of collecting droplets of first type under conditions that temporarily inactivate said nuclease before step (e).

In yet another embodiment, the conditions of step (b) comprise selecting a temperature degree ranging from -20°C to 10°C and condition of step (c) comprise selecting a temperature degree ranging from 20°C to 40°C.

Droplets can be incubated outside the microfluidic system (off-chip) for single-cell chromatin fragmentation. As the lysis occurs in droplets, the nuclear DNA from lysed cells is accessible for nuclease enzyme. Therefore, the kinetic of the digestion is particularly important to yield preferentially mono-nucleosomes, which are retained in the droplet.

In a particular embodiment, the incubation step (c) is timed to obtain nuclear DNA fragmented into mono-nucleosomes.

In yet another embodiment, the one or more genomic region(s) of interest is a modified genomic region.

In yet another embodiment, the modified genomic region comprises a protein complex associated with a nucleic acid sequence and/or a nucleic acid sequence. In a particular embodiment, the modified genomic region is a modified mono-nucleosome. In another embodiment, the modified genomic region is a transcription factor binding site, a chromatin modifier binding site, a chromatin remodeler site, a histone chaperone binding site.

In yet another embodiment, the modified genomic region comprises a post-translational modification selected from the group comprising acetylation, amidation, deamidation, carboxylation, disulfide bond, formylation, glycosylation, hydroxylation, methylation, myristoylation, nitrosylation, phosphorylation, prenylation, ribosylation, sulphation, sumoylation, ubiquitination and derivatives thereof.

In yet another embodiment, the modified genomic region comprises a modified DNA sequence selected from the group comprising methylation and its derivatives, modified nucleotides like EdU, BrdU, IdU, CldU and others. The most common way of modifying bases is the addition of a methyl mark, and across species, methylation has been found on cytosines and adenines, resulting in 5mC, N4-methylcytosine (N4mC), or 6-methyladenine (6mA), 5-hydroxymethylcytosine (5hmC), 5-formylcytosine (5fC) and 5-carboxylcytosine (5caC).

As introduced above, a remarkable limitation of the method disclosed in Rotem is that only symmetrically indexed nucleosomes can be amplified and can be part of the sequencing library. This requirement dramatically increases the stringency of the system and imposes a strong selection on the nucleosomes, which are limited to those with both ends ligated to a barcode. In contrast with the Drop-ChIP method, the inventors have surprisingly found that indexing nucleosomes from only one end would increase the single-cell coverage and ultimately the capacity of the system to distinguish more subtle variations between single-cell chromatin profiles.

In yet another embodiment, the nucleic acid sequence is asymmetrically linked to said at least one or more genomic region(s) of interest.

As used herein, the term "asymmetrically linked" refers to the presence of at least one barcode linked to a genomic region of interest, whereby the linkage is to only one of the two extremities of the genomic region of interest.

In a second aspect of the present invention, there is provided a nucleic acid sequence comprising:
a. at least one index sequence,
b. a sequencing adaptor, and
c. at least one protecting function positioned at 3'- and/or 5'-end.

As used herein, the term "nucleic acid sequence" refers to a single- or double-stranded nucleic acid. In yet another embodiment, a "nucleic acid sequence" can be DNA or RNA. In a preferred embodiment, the "nucleic acid sequence" is a double-stranded DNA. In some embodiments, the "nucleic acid sequence" comprises a double-stranded DNA comprising a first strand barcode and a second strand barcode. In some embodiments, the first strand barcode and second strand barcode comprise complementary sequences. In some embodiments, the first strand barcode and second strand barcode comprise non-complementary sequences.

As used herein, the term "index sequence" refers to a unique nucleotide sequence that is distinguishable from any other index sequences as well as from any other nucleotide sequences within the nucleic acid sequence, wherein it is comprised. An "index sequence" can be a random or a specifically designed nucleotide sequence. An "index sequence" can be of any sequence length. A nucleic acid sequence according to the second aspect of the present invention, can be attached to a genomic region of interest (target) to tag a species that requires to be identified and/or to discriminate different members of the tagged species within a population thereof. Accordingly, in the context of the present invention, the terms "index sequence" and "barcode" can be used interchangeably.

As used herein, the term "sequencing adapter" refers to an oligonucleotide of known sequence, the ligation or incorporation of which to a polynucleotide or a polynucleotide strand of interest enables the generation of products of said polynucleotide or said polynucleotide strand of interest ready for amplification.

In one embodiment of the second aspect of the invention, the nucleic acid sequence further comprises at least one cleavage site.

As used herein, the term "cleavage site" refers to a target region of the nucleic acid sequence that is susceptible of being cleaved by any means, including but not limited to enzymes, that is capable of cleaving a single or double-stranded nucleic acid sequence. In the context of the present invention, the "cleavage site" can serve to cleave or otherwise release a portion of the nucleic acid sequence. The "cleavage site" is recognized by a cleaving agent, which can be natural, synthetic, unmodified or modified.

In one embodiment of the present invention, the protecting function is selected from the group comprising a spacing element on a 3'-end and a dideoxy-modified base on 5'-end. In the context of the present invention, a suitable non-limiting spacing element is a three carbon spacer (C3 spacer).

In yet another embodiment of the present invention, the at least one cleavage site is a restriction site comprising a palindromic region.

As used herein, the term "restriction site" refers to a site that is recognized by a restriction enzyme, e.g. an endonuclease. A person skilled in the art is familiar with restriction endonucleases and their restriction sites. Non-limiting examples of restriction site include BamHI, BsrI, Notl, Xmal, PspAI, Dpnl, Mbol, MnlI, Eco57I, Ksp632I, DraIII, AhaII, Smal, MluI, HpaI, Apal, BclI, BstEII, TaqI, EcoRI, SacI, HindII, HaeII, DraII, Tsp509I, Sau3AI, PacI.

In yet another embodiment of the present invention, the nucleic acid sequence is suitable for use in a method according to the first aspect of the present invention and its embodiments.

In another embodiment of the present invention, the nucleic acid sequence is suitable for use in profiling the epigenetic state in a sample obtained from a subject.

As used herein, the term "sample" refers to a biological sample.

As used herein, the term "subject" refers to a human or a non-human subject. In the latter case, the non-human subject is not limited to a mammal subject.

The method according to the present invention can find different application in the identification of genes, and factors involved in the diagnosis and/or prognosis of a diseased state in a subject, as well as methods for the diagnosis and/or prognosis of a diseased state in a subject and for controlling the effect of therapeutics molecules on chromatin.

In the context of the present invention, a diseased state can refer to any modification involving nucleosomes or nucleic acid sequences, as well as positioning of proteins impacting chromatin structure, regulation and function. As used herein, the expression "diseased state" also encompasses an abnormal rate of cell proliferation so that the treatment of the disease requires modulation of the cell cycle. Examples of proliferative diseases include, without being limited thereto, cancer.

### EXAMPLES

### Microfluidic workflow of the single-cell ChIP-seq procedure

A general scheme of the microfluidic method according to the present invention is depicted in Figure 1. (a) Cells are compartmentalized in 45pl droplets with the reagents necessary for cell lysis and chromatin fragmentation. In parallel, hydrogel beads carrying DNA barcodes are encapsulated in 100pl droplets with ligation reagents and EGTA that inactivates MNase. The two emulsions are re-injected in a fusion device, the barcode-containing droplets (100pl) and the nucleosome-containing droplets (45pl) are paired asymmetrically and merged by electro-coalescence triggered by an electric field. Fused droplets are scanned one-by-one by a laser beam to analyze the composition of each droplet in real time. (b) Emulsion of fused droplets is collected and incubated off-chip for nucleosomes barcoding in droplets. Barcodes are released from the beads by photo-cleavage and ligated to the nucleosomes. The content of droplets is combined, immunoprecipitated and the enriched DNA is sequenced. The deconvolution of the barcode-associated reads attributes all sequences to their originating cell in order to reconstruct single-cell chromatin profile.

### Synchronizing and pausing chromatin fragmentation in droplets

Cells are compartmentalized in 45pl droplets with a digestion mix comprising the lysis buffer and the MNase (see Figure 1). After complete lysis, chromatin is released into the droplets and accessible to get cleaved by the MNase. This section presents a typical calibration of MNase activity in droplets in order to yield preferentially fragments of the size of a nucleosome. However, performing enzymatic assays in droplets might be challenging as cells are processed individually with different time scales. Consequently, fine-tuning enzymatic activity is necessary to avoid chromatin digestion discrepancy between droplets and single-cells.

### Compartmentalization of cells in droplets

The number of cells per droplet follows a Poisson distribution, which describes the probability of finding a mean number λ of x cells per droplet (Howard Shapiro, Pratical Flow Cytometry, 4th edition. Wiley-Liss, 2003). In single-cell ChlP-seq experiment, cell density is adjusted to encapsulate λ = 0.1 cells in 45pl droplets, resulting in 90.5% of empty droplets, 9% of droplets containing one single cell, 0.5% containing two cells and 0.015% containing more than two cells. Real-time monitoring of the compartmentalization of cells in droplets is performed by pre-labelling the cells with Calcein AM, a non-fluorescent derivate of calcein. Upon entering into the cells, the acetomethoxy group (AM) gets cleaved by intracellular esterase and releases a strong green fluorescence (excitation/emission: 495/515nm). The fluorescence is acquired as the droplets crossed the laser beam at the detection point, allowing counting the number of cells encapsulated.

### Droplets collection

Droplets are collected in a collection tube on ice until the end of the encapsulation (10min to 20min depending on the number of starting cells). After encapsulation, drops are incubated at 37°C for MNase digestion.

### MNase calibration in droplets

At the end of the encapsulation, droplets are incubated off-chip for single-cell chromatin fragmentation. Cells are lysed in droplets, making their nuclear DNA accessible for MNase enzyme. The kinetic of the digestion is particularly important to yield preferentially mono-nucleosomes, which are retained in the droplet. The ideal incubation time is defined as the time necessary to get 100% of nuclear DNA fragmented into mono-nucleosomes. The digestion conditions including lysis buffer composition, MNase concentration and incubation time are precisely calibrated for each sample by performing a time-course study. The calibration is carried out as follows: 45pl droplets containing cells, lysis buffer and MNase are produced, collected in a collection tube and placed at 37°C for different incubation time. At each time point, a fraction of droplets is broken and MNase immediately inactivated by addition of EGTA (see Figure 3). DNA fragments are then purified and analyzed by electrophoresis. The choice of the incubation time is a balance between having the highest proportion of mono-nucleosomes but, in the same time, preventing nucleosomal DNA to be over-digested. Indeed, it was hypothesized that DNA protruding from the nucleosome should be long enough to enable an efficient ligation of the barcodes in the subsequent steps of the procedure.

### Controlling MNase activity in droplets

MNase activity in droplets is controlled by collecting the droplets on ice upon cell encapsulation (see Figure 2). Indeed, the time point t = 0min in Figure 2, which corresponded to a fraction of droplets taken at the end of the droplets production but just before incubation, indicates that nuclear DNA is not digested yet by MNase. This evidence confirms that chromatin digestion does not occur upon droplet production but starts immediately with incubation at 37°C (see Figure 2).

Placing the droplets on ice after incubation and upon re-injection in the fusion device may "pause" MNase activity and limit cell-to-cell variation in chromatin digestion. For this purpose, two droplet fractions are taken after 12min MNase incubation: one fraction is immediately processed to control for digestion, while the second fraction is stored beforehand for 1h on ice, then processed similarly. As expected, the time points t = 12min and t = 12min + 1h ice on Figure 2 confirm that MNase is no longer active in the fraction stored on ice (as compared with t = 20min time point). Consequently, storing droplets on ice "pauses" MNase activity, thus preventing again variation in chromatin digestion between droplets

### DNA barcoding strategy

DNA barcodes are grafted to the hydrogel beads via a streptavidin-biotin linkage and a photo-cleavable moiety enabling release from the beads when exposed to UV light (Klein et al. 2015, Cell 161(5):1187-1201). The synthesis of barcodes consists in distributing the beads in a microtiter well plate containing ligation reagents and 96 combinations of a 20 bp oligonucleotide (later referred to as Index 1). Index 1 are ligated to the beads and the latter are pooled before being distributed again in a second microtiter plate containing 96 new combinations of a 20 bp oligo (later referred to as Index 2). By repeating 3 times this split-pool method, a library of 96³ possible barcode combinations is easily generated (i.e. 884,736 combinations).

### Quality controls of barcoded hydrogel beads

Barcoded beads are one of the core reagents of the scChlP-seq technology, their quality have been systematically controlled to ensure that cell-to-cell variations originate from true biological differences in their histone modification patterns rather than technical artefacts.

Tapestation profile of DNA barcodes released from the beads revealed that >75% were full length (larger peak 146 bp), as well as the presence of intermediates that failed to be completed (Figure 5). In average, the number of full-length barcodes is estimated to 5x10⁷ copies per barcoded hydrogel bead.

To validate the release of barcodes from the hydrogel beads, DNA probes are hybridized to the barcodes onto the beads. The latter are then encapsulated in 100pl droplets and collected off-chip as in a scChIP-seq experiment. Part of the droplets are re-injected as a single file into a micrometric chamber as reported by Eyer (Eyer et a/. 2017, Nature Biotechnology 35(10):977-982) and imaged the beads by epifluorescence microscopy while the fluorescent barcodes are still bound on the beads. As expected, the fluorescence is localized on the beads (see Figure 5). A second fraction of beads-containing droplets is exposed to UV light to initiate barcodes release. As described above, epifluorescence microscopy of bead-containing droplets after photo-cleavage reveals a uniform distribution of the fluorescence in the droplets, suggesting complete barcode release (see Figure 5). Lastly, single-bead sequencing is performed on every new batch of barcoded beads. Beads are isolated by limiting dilution in a 384-well plate. Only wells containing one bead are selected by imaging for amplification and sequencing of the barcodes. The analysis of the sequencing data shows the fraction of the first two most abundant barcodes of 16 beads. Hundred thousand of different barcodes are identified per bead but, in average, the most abundant barcode represents 97.7% of the sequencing reads. The second most abundant barcode takes on average as few as 0.17% of the reads, suggesting that all the other identified barcodes are negligible (see Figure 5).

### Barcode design

Barcodes are bound to the beads via streptavidin-biotin linkages, which are in turn separated from the 5'-end of the oligonucleotide by photo-cleavable entities. The latter are composed of the photo-cleavable group as well as an alkyl spacer that minimize steric interactions (the all entity is referred to as PC-linker, see Figure 4). The first biotinylated and PC-linker oligonucleotide is common to all barcodes and comprises a T7 promoter sequence and an Illumina sequencing adaptor (SBS12 sequence). The T7 promoter sequence serves as a recognition site for the T7 RNA polymerase to initiate the linear amplification of enriched barcoded nucleosomes post-immunoprecipitation in an *in-vitro* transcription reaction (IVT). This amplification strategy is widely adopted for unbiased, sensitive and reproducible amplification of cDNAs post-reverse transcription in single-cell RNA-seq protocols (Hashimshony et al. 2012, Cell Reports 2(3):666-673). In a second step, the Illumina sequencing adaptor serves as PCR handle to complete the preparation of the sequencing library. Also, this adaptor is necessary for the Next Generation Sequencing of the samples as the primer that initiates Read #2 and the reading of the barcode sequence. The beads grafted with this first common oligonucleotide are then used for barcode synthesis by successive ligation of the 3 indexes. Unfortunately, the analysis of a first single-cell ChlP-seq dataset reveals that only few reads (∼38%) have a complete and correct barcode structure.

An optimized barcode structure allowing the digestion of barcode concatemers as well as reducing the ligation of non-full-length barcodes is depicted in Figure 3. Barcodes are framed with one half of Pac1 restriction site, which is only reconstructed in case of formation of concatemers. Those are digested after the immunoprecipitation but before the linear amplification to clean-up the library. Barcodes photo-cleaved side is modified with the incorporation of a 3' C3-spacer. This modification introduces a spacer arm at the 3'-hydroxyl group of the 3' base and blocks the ligation. With the addition of the spacer group, the direction of the ligation is enforced from the 3'-end of the barcodes to the nucleosomes. Non-full-length barcodes are completed with a "block" oligonucleotide sequence comprising a 3' C3-spacer and a 5' inverted dideoxy-T base. Again, both modifications aim at limiting unwanted ligation events.

### Encapsulation of barcoded beads in droplets

Loading discrete objects such as hydrogel beads into droplets can be estimated by a Poisson distribution. In the same way as the encapsulation of the cells, the loading of the beads is monitored in real time as the droplets crossed the laser beam at the detection point. In single-cell ChIP-seq experiment, it is typically achieved between 65% to 75% of droplets that contain a barcoded hydrogel bead.

### Merging nucleosomes-containing droplets with barcodes-containing droplets

Cells and DNA barcodes are separately encapsulated to prevent barcodes digestion by MNase. To index chromatin at the single-cell level, DNA barcodes have to be delivered in a second step into nucleosomes-containing droplets. This is achieved by active fusion of the two droplets populations in a dedicated microfluidic device using a triggered electric field.

Droplets from the "cell emulsion" and droplets from the "barcode emulsion" are re-injected as a single-file in the microfluidic fusion device. Achieving proper electro-coalescence requires one-to-one pairing of the droplets from the two emulsions. Hydrodynamic forces enable the faster smaller 45pl droplets ("cell emulsion") to catch up and come in contact with the 100pl droplets ("barcode emulsion"), as contact is necessary for the two droplets to fuse (Mazutis et al. 2009, Lab on a Chip 9(18):2665). Similarly to droplet productions, fluorescence intensity of fused droplets is acquired as they crossed the laser beam at the detection point (see Figure 1).

### Reconstructing cell type-specific chromatin states from single-cell ChIP-seq profiles

Human T lymphocytes and human B lymphocytes are separately encapsulated and indexed with two distinct sets of barcodes as shown in Figure 7. After nucleosomes barcoding in droplets, indexed chromatin from the two cell types are combined, performed chromatin immuno-precipitation and sequenced the library

Introduction of bias related to the immuno-precipitation or preparation of the sequencing libraries (batch effect) is avoided by combining indexed chromatin. Each sequencing read would carry a double information: (1) the single-cell barcode sequence, which assigns the read to its cell of origin; (2) the "cell-type specific sequence", which assigns the read to one cell type (either B or T lymphocyte).

### Deconvolution of single-cell barcodes and cell type-specific sequences

Barcodes were extracted from Reads #2 by first searching for the constant 4 bp linkers found between the 20-mer indices of the barcode allowing up to 1 mismatch in each linker. If the correct linkers were identified, the three interspersed 20-mer indices were extracted and concatenated together to form a 60 bp non-redundant barcode sequence. A library of all 884,736 combinations of the 3 sets of 96 indices (96³) was used to map barcode sequences using the sensitive read mapper Cushaw3. Each set of indices is error-correcting because it takes more than an edit-distance of 3 to convert one index into another. We therefore set a total mismatch threshold of 3 across the entire barcode, with two or less per index to avoid mis-assigning sequences to the wrong barcode Id. In a second, slower step, sequences that could not be mapped to the Cushaw3 index-library were split into their individual indices and each index compared against the set of 96 possible indices, allowing up to 2 mismatches in each individual index. Any sequences not assigned to a barcode Id by these two steps were discarded.

## Claims

1. A method of identifying one or more genomic region(s) of interest using a microfluidic system, the method comprising the steps of:
a. providing at least a droplet of first type, wherein said droplet of first type comprises
i. a biological element,
ii. a lysis buffer, and
iii. a nuclease,
b. collecting said droplet of first type under conditions that temporarily inactivate said nuclease,
c. incubating said droplet of first type, thereby reactivating said nuclease,
d. providing at least a droplet of second type, wherein said droplet of second type comprises a nucleic acid sequence,
e. merging said droplets of first and second type, thereby generating a droplet of third type,
f. incubating said droplet of third type, thereby linking said nucleic acid sequence to at least one genomic region(s) of interest,
g. sequencing said one or more genomic region(s) of interest.

2. The method according to claim 1, wherein the conditions of step (b) comprise selecting a temperature degree ranging from -20°C to 10°C and condition of step (c) comprise selecting a temperature degree ranging from 20°C to 40°C.

3. The method according to any of the claims 1 to 2, wherein the one or more genomic region(s) of interest is a modified genomic region.

4. The method according to claim 3, wherein the modified genomic region comprises a protein complex associated with a nucleic acid sequence and/or a nucleic acid sequence.

5. The method according to any of the claims 3 and 4, wherein the modified genomic region comprises a post-translational modification selected from the group comprising acetylation, amidation, deamidation, carboxylation, disulfide bond, formylation, glycosylation, hydroxylation, methylation, myristoylation, nitrosylation, phosphorylation, prenylation, ribosylation, sulphation, sumoylation, ubiquitination and derivatives thereof.

6. The method according to any of the claims 1 to 5, wherein the nucleic acid sequence is asymmetrically linked to said at least one or more genomic region(s) of interest.

7. A nucleic acid sequence comprising:
a. at least one index sequence,
b. a sequencing adaptor, and
c. at least one protecting function positioned at 3'- and/or 5'-end.

8. The nucleic acid sequence according to claim 7, wherein said nucleic acid sequence further comprises at least one cleavage site.

9. The nucleic acid sequence according to any of the claims 7 and 8, wherein the protecting function is selected from the group comprising a spacing element on a 3'-end and a dideoxy-modified base on 5'-end, or inversely.

10. The nucleic acid sequence according to claims 7 to 9, wherein the at least one cleavage site is a restriction site comprising a palindromic region.

11. The nucleic acid sequence according to any of the claims 7 to 10 for use in a method according to any of the claims 1 to 6.

12. The nucleic acid sequence according to any of the claims 7 to 11 for use in profiling the epigenetic state in a sample obtained from a subject.
